# EUROPEAN PATENT APPLICATION

(11) **EP 4 089 392 A1**
(43) Date of publication of application: **16.11.2022**
(21) Application number: 21173801.8
(22) Date of filing: 14.05.2021
(51) Int. Cl.: G01N 1/40, G01N 33/02, G01N 1/44

(54) **EXTRACTION METHODS FOR FOOD ALLERGENS**

(71) Applicant: Fraunhofer-Gesellschaft zur Förderung der angewandten Forschung e.V., 80686 München (DE)
(72) Inventor: UEBERHAM, Elke, 80686 München (DE); LIDZBA, Norbert, 80686 München (DE)
(74) Representative: Kuttenkeuler, David

(57) **Abstract**

The present invention pertains to methods for extracting an allergen from a sample, in particular a food allergen from a food sample. The present invention also relates to methods for detecting an allergen in a sample. The present invention makes use of an ionic detergent having a steroid scaffold for the extraction of the allergen from the sample. Preferably, the ionic detergent for use in the extraction of an allergen from a sample having a steroid scaffold is a bile acid, a bile acid derivative, a bile acid analog, or a salt thereof. Particularly preferred is the use of sodium deoxycholate, or a derivate thereof, as ionic detergent for the extraction of allergens. Kits for performing the above methods are also provided.

## Description

### FIELD OF THE INVENTION

The present invention pertains to methods for extracting an allergen from a sample, in particular a food allergen from a food sample. The present invention also relates to methods for detecting an allergen in a sample. The present invention makes use of an ionic detergent having a steroid scaffold for the extraction of the allergen from the sample. Preferably, the ionic detergent for use in the extraction of an allergen from a sample having a steroid scaffold is a bile acid, a bile acid derivative, a bile acid analog, or a salt thereof. Particularly preferred is the use of sodium deoxycholate, or a derivate thereof, as ionic detergent for the extraction of allergens. Kits for performing the above methods are also provided.

### DESCRIPTION

Food allergy is an emerging major public health problem, and a wide variety of different kinds of foods can contain allergens, contaminants, or toxins. Examples of food allergies include gluten allergy, dairy allergy, fish allergy, nut allergy, soy allergy, fruit allergy, and many more. Food allergy can cause a detrimental immunoreaction in consumers, which is the result of ingestion of an allergy-inducing substance in a food (referred to as an allergen, or food allergen in the context of the present invention). The food allergy can, for example, cause dermatitis, asthma, mucus secretion, nerve stimulation, smooth muscle contraction, digestive-tract obstacle, or anaphylaxis shock.

Allergies can have a significant impact on psychosocial aspects of quality of life extending beyond the immediate clinical effects of the patient's allergic condition and the daily activities of families. At present, the standard of care for this type of allergy includes strict avoidance of ingestion of food containing allergens for susceptible consumers. If food is ingested by susceptible consumers by accident, treatment with epinephrine might be necessary to prohibit detrimental consequences for the consumer.

One example of a common food allergy is peanut allergy, which is an immune response to dietary substances from peanuts causing an overreaction of the immune system. The European Center for Allergy Research Foundation (ECARF) estimates that 0.2 per cent of the entire European population is allergic to peanuts, and peanut allergy is the most common cause of food-related death. Tree nuts such as pecans, pistachios, pine nuts and walnut are also common nut allergens.

In general, allergies are classified into types I to IV, depending on the mechanism of disease onset. Food allergy is primarily a type I hypersensitivity (IgE mediated), in which excessive IgE antibodies react with food allergens within the body, and cause the inflammatory response. To prevent an allergic reaction to a food allergen, it is necessary that susceptible consumers prevent ingestion of food containing allergens. Accordingly, it is necessary to provide information to the consumers through adequate labeling of the food, so as to allow lowering or preventing the risk of an allergic reaction to food allergens in consumers.

Food labelling requirements are laid down in EU legislation. The EU Regulation No. 1169/2011 has been implemented in all EU member states on 13 December 2014, and uniform standards apply throughout all the member states of the European Union. According to the EU Regulation No. 1169/2011, manufacturers are obliged to disclose a range of clearly legible information on the label - including details about the ingredients in a clearly visible way. Such labels with information about the ingredients and properties of a consumable serves to protect consumers and assist them in making better-informed choices. Similar international regulations oblige manufactures to disclose details about the ingredients of food that is sold outside of the EU.

Accordingly, an enormous amount of effort is being made by manufacturers as well as certain agencies to analyze the contents of food, e.g. for the presence of food allergens. These methods largely depend on the reliable extraction of allergens from samples, such as food samples, in a soluble state. Importantly, the soluble state of allergens is the requirement for analysis of the presence of allergens in a sample, e.g. by antibody-based methods and/or mass spectrometry.

WO2011098569 A1 discloses processes for producing native, depigmented and polymerised allergen extracts. The invention further discloses extracts produced via the processes, and pharmaceutical and vaccine compositions comprising the extracts, for diagnosis and treatment of allergy.

WO03022876 A1 provides a method for detecting food allergens, antibodies and antigens to prepare the antibodies.

WO2015017442 A2 discloses methods and devices for the detection of food allergens using molecularly imprinted polymers that are imprinted for a target food allergen.

However, there is still a huge need for reliable methods to detect allergens in food, and none of the prior art methods can provide the extraction of allergens from samples in their physiological configuration. In view of the above, there is a continuing need in the art to provide an efficient method for extracting an allergen from a sample. There is a further need to provide a sensitive method for detecting an allergen in a sample. A preferred tool for extraction of allergens will have several fundamental characteristics. It should be universally applicable to all different kinds of allergens, which can be derived from any kind of sample, preferably a food sample, and should be easy and fast to use. The tool should further allow the subsequent reliable and sensitive detection of the allergen.

The invention solves the above problems by providing a novel method for extracting an allergen from a sample, preferably a food sample. The method is based on using an extraction solution comprising an ionic detergent having a steroid scaffold.

### BRIEF DESCRIPTION OF THE INVENTION

Generally, and by way of brief description, the main aspects of the present invention can be described as follows:
In **a first aspect,** the invention pertains to a method for extracting an allergen from a sample.
In **a second aspect,** the invention pertains to the use of an ionic detergent having a steroid scaffold for the extraction of an allergen from a sample.
In **a third aspect,** the invention pertains to a method for detecting an allergen in a sample.
In **a fourth aspect,** the invention pertains to a kit for performing a method according to the present invention.

### DETAILED DESCRIPTION OF THE INVENTION

In the following, the elements of the invention will be described. These elements are listed with specific embodiments, however, it should be understood that they maybe combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In the first aspect, the invention pertains to a method for extracting an allergen from a sample, the method comprising the steps of:
a) Providing a sample, and an extraction solution comprising an ionic detergent having a steroid scaffold, and
b) Contacting the sample with the extraction solution,
thereby extracting the allergen from the sample.

The inventors surprisingly found that an extraction solution comprising an ionic detergent having a steroid scaffold, such as a bile acid, a bile acid derivative, a bile acid analog, or a salt thereof, is very effective in solubilizing and stabilizing an allergen during extraction. Bile acids are steroid acids found predominantly in the bile of mammals and other vertebrates. Bile acids are often conjugated with taurine or glycine residues to give anions called bile salts. Primary bile acids are synthesized by the liver, whereas secondary bile acids result from bacterial actions in the colon. As such, the invention makes use of the emulsifying and solubilizing characteristics of bile acids and/or bile acid salts, which physiologically solubilize ingredients within the digestive tract.

The inventors discovered that the presence of an ionic detergent having a steroid scaffold, such as a bile acid, a bile acid derivative, a bile acid analog, or a salt thereof, partly unfolds the allergen and makes it accessible for further detection reagents, such as antibodies used for detection of the allergen. In a partly unfolded state, an allergen is better accessible for the entry of antibodies, which allows a more efficient binding of the antibodies to the allergen to be detected. Accordingly, the detection of the allergen is easier and more reliable if extracted using an extraction solution comprising an ionic detergent having a steroid scaffold, such as a bile acid, a bile acid derivative, a bile acid analog, or a salt thereof.

It is also an advantage of the present invention that the extraction process is similar to physiological digestion within a human's body. Accordingly, allergens extracted using the extraction method of the present invention have a physiological configuration, which is highly advantageous for further detection methods since allergens are in a state that would normally cause an allergic reaction within the consumer's body. Importantly, epitopes present within the allergen become accessible in presence of bile acid salts. This is true both during physiological digestion, and also when using *in vitro* extraction methods according to the present invention. Such epitopes are of great interest, since they can be the cause of an allergic reaction within the human body. Other methods, such as those that rely on, e.g., addition of sodium dodecyl sulfate (SDS), extract allergens in a very artificial state that often largely differs from its physiological configuration. Accordingly, detection of epitopes of an allergen in an extraction solution comprising SDS can also largely differ from the presence of epitopes of allergens within the human's body.

In a preferred embodiment, the ionic detergent having a steroid scaffold is a bile acid, a bile acid derivative, a bile acid analog, or a salt thereof.

In one embodiment, the ionic detergent having a steroid scaffold comprises cis-fused rings A and B in the steroid scaffold, i.e. a cis-junction between rings A and B of the steroid nucleus. Preferably, the steroid scaffold according to the present invention also has an isopentanoic lateral chain. The detergent having a steroid scaffold can further comprise one, two, three, four, or any other number of hydroxyl groups, which are preferably located at positions 3, 6, 7, and 12. Ionic detergents having a steroid scaffold according to the present invention can differ in the number, position and stereochemistry of hydroxyl groups.

Further preferred is that the bile acid, the bile acid derivative, the bile acid analog, or the salt thereof is cholic acid, glycocholic acid, taurocholic acid, chenodeoxycholic acid, glycochenodeoxycholic acid, taurochenodeoxycholic acid, deoxycholate, sodium deoxycholate, deoxycholic acid, cholate, sodium cholate, deoxycholate, chenodeoxycholate, chenodeoxy cholic acid, ursodeoxycholic acid (UDCA), dehydrocholic acid, or sodium dehydrocholate, preferably wherein the ionic detergent is sodium deoxycholate, or a derivate thereof.

Particularly preferred is that the ionic detergent is an anionic detergent. In a particularly preferred embodiment, any derivative of cholic acid in its anionic form and having amphiphatic character is particularly useful as ionic detergent in the sense of the present invention. Particularly preferred for extraction of an allergen is an ionic detergent having amphiphatic character, since the amphiphatic character enables a very efficient solubilization and stabilization of the allergen to be extracted from the sample.

One embodiment relates to the method of the first aspect of the present invention, wherein the concentration of the ionic detergent having a steroid scaffold in the extraction solution is at least 0.01% (w/v) of the extraction solution, preferably at least 0.1% (w/v), and most preferably at least 1% (w/v) of the extraction solution.

In another preferred embodiment, the extraction solution further comprises a buffer, and wherein the pH of the extraction solution is between 7.0 - 10.0, preferably between 8.0 - 9.0, and most preferably around 8.5.

According to the present invention, any kind of buffer can be used. Preferably, the buffer is a tris buffer, phosphate buffer, carbonate buffer, sodium phosphate buffer, glycine sodium hydroxide buffer, sodium hydrogen phosphate buffer, sodium dihydrogen phosphate buffer, potassium phosphate buffer, potassium hydrogen phosphate buffer, potassium dihydrogen phosphate buffer, pyrophosphate buffer, sodium pyrophosphate/hydrochloric acid buffer, sodium carbonate buffer, potassium carbonate buffer, sodium hydrogen carbonate buffer, and/or potassium hydrogen carbonate buffer, more preferably wherein the buffer is a 10-100 mM tris buffer, even more preferably a 30-80 mM tris buffer, even more preferably a 40-60 mM tris buffer, and most preferably a 50 mM tris buffer.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure. In particularly preferred embodiments of the invention, the term "about" may refer to a deviation of the respective numeric value of a maximum of 20% of the numerical value, however more preferred is 15%, 10%, 5%, even more preferred is 4%, 3%, 2%, and most preferred is 1%.

Yet another embodiment pertains to the method of the first aspect of the present invention, wherein the extraction solution has an ionic strength corresponding to a sodium chloride concentration of at least 200 mM, preferably corresponding to a sodium chloride concentration of at least 300 mM, and most preferably corresponding to a sodium chloride concentration of around 400 mM. However, the presence of sodium chloride in the extraction solution is no necessity for the present invention. The extraction solution may also allow an efficient extraction of allergens without presence of an additional salt, such as sodium chloride. Alternatively, any kind of salt may be used instead of sodium chloride in case an extraction solution having an ionic strength corresponding to a sodium chloride concentration of at least 200 mM is preferred, such as potassium chloride, or the like.

In one embodiment, the contacting in step b) of the method is performed at a temperature of between 80°C and 120°C, preferably between 90°C and 110°C, more preferably between 95°C and 105°C, and most preferably at around 99°C. In a further preferred embodiment, the contacting in step b) of the method is performed for a duration of at least 10 min, preferably for at least 20 min, more preferably around 20 to 30 min.

The present invention enables extraction of any kind of allergen. In one embodiment, the allergen is a food allergen, preferably wherein the food allergen is a nut or legume allergen (e.g., from peanut, walnut, almond, pecan, cashew, hazelnut/filbert, pistachio, pine nut, or brazil nut), a dairy allergen (e.g., from egg, or milk), a seed allergen (e.g., from sesame, poppy, or mustard), a seafood allergen (e.g., from shrimp, fish, crab, lobster, clams, mussels, oysters, scallops, crayfish, or shellfish), soybean, wheat, or a fruit allergen (e.g., from strawberries, mango, banana, or apple). One example of a food allergen to be extracted from egg is ovalbumin.

According to the present invention, any kind of allergen can be extracted from any kind of sample. In one embodiment, the sample is a biological allergen source material, such as a food sample, a sample derived from the environment, such as a sample from soil or from seawater, from at least one organism such as from at least one bacterium, fungus, protist, algae, plant, an animal, or a mixture thereof. Without being limited thereto, examples of food samples according to the present invention are nuts (such as peanuts, walnuts, almonds, pecans, cashews, hazelnuts, pistachios, pine nuts, or brazil nuts), eggs, milk, seeds (such as sesame, poppy, or mustard), seafood (such as shrimp, fish, crab, lobster, clams, mussels, oysters, scallops, crayfish, or shellfish), soybean, wheat, or fruits (such as strawberries, mango, banana, or apple). Importantly, the extraction solution of the present invention is able to extract allergens even from very fatty samples, such as peanut-containing food samples.

The term "at least one" according to the present invention shall include 1, 2, 3, 4, 5, 6, 7, 8, 9, 10, 11, 12, 13, 14, 15, 16, 17, 18, 19, 20, or any other number. For example, the term "at least one" organism shall include 1, 2, 3, 4, 5, 6, 7, 8, 9, 10, 11, 12, 13, 14, 15, 16, 17, 18, 19, 20, or any other number of organisms. Similarly, the term "at least one" plant shall include 1, 2, 3, 4, 5, 6, 7, 8, 9, 10, 11, 12, 13, 14, 15, 16, 17, 18, 19, 20, or any other number of plants.

One embodiment relates to the above method of the first aspect, wherein if the allergen is a nut or legume allergen or soy, the extraction solution has an ionic strength corresponding to a sodium chloride concentration of at least 350 mM, preferably corresponding to a sodium chloride concentration of around 400 mM.

An ionic strength corresponding to a sodium chloride concentration of at least 350 mM is particularly preferred if the allergen is a nut or legume allergen (e.g. peanut), or soy, because globulins are salt-soluble. However, many other allergens are also salt-soluble. Accordingly, it may be preferred that the extraction solution has an ionic strength corresponding to a sodium chloride concentration of at least 350 mM for any allergen to be extracted from a sample, to enable an efficient extraction from the sample. It is particularly preferred that the extraction solution has an ionic strength corresponding to a sodium chloride concentration of at least 350 mM for any allergen in globulin form to be extracted from a sample.

A further embodiment relates to the above method of the first aspect, wherein if the allergen comprises at least one disulfide bond, preferably more than one disulfide bonds, the extraction solution further comprises a reducing agent, such as Tris(2-carboxyethyl)phosphine (TCEP). Without being limited thereto, a further reducing agent that may be used is thioglycerol.

In one embodiment, the extraction solution does not comprise sodium dodecyl sulfate (SDS).

In a further embodiment, the extraction solution comprises 50 mM Tris pH 8.5, 400 mM sodium chloride, and 1% (w/v) sodium deoxycholate, or a derivate thereof.

The methods of the first aspect of this invention, are preferably *ex-vivo* or *in-vitro* methods.

The present invention also relates to a food extract having been extracted with a method according to the first aspect of this invention.

A second aspect of the present invention, which can be combined with any other aspect and specific embodiment of the present invention, pertains to the use of an ionic detergent having a steroid scaffold, wherein the ionic detergent having a steroid scaffold is a bile acid, a bile acid derivative, a bile acid analog, or a salt thereof, for the extraction of an allergen from a sample.

In a preferred embodiment of the second aspect of the present invention, the ionic detergent having a steroid scaffold is a bile acid, a bile acid derivative, a bile acid analog, or a salt thereof.

Further preferred is that the bile acid, the bile acid derivative, the bile acid analog, or the salt thereof is cholic acid, glycocholic acid, taurocholic acid, chenodeoxycholic acid, glycochenodeoxycholic acid, taurochenodeoxycholic acid, deoxycholate, sodium deoxycholate, deoxycholic acid, cholate, sodium cholate, deoxycholate, chenodeoxycholate, chenodeoxy cholic acid, ursodeoxycholic acid (UDCA), dehydrocholic acid, or sodium dehydrocholate, preferably wherein the ionic detergent is sodium deoxycholate, or a derivate thereof.

One embodiment relates to the use of the second aspect of the present invention, wherein the concentration of the ionic detergent having a steroid scaffold in the extraction solution is at least 0.01% (w/v) of the extraction solution, preferably at least 0.1% (w/v), and most preferably at least 1% (w/v) of the extraction solution.

In another preferred embodiment, the extraction solution further comprises a buffer, and wherein the pH of the extraction solution is between 7.0 - 10.0, preferably between 8.0 - 9.0, and most preferably around 8.5.

According to the present invention, any kind of buffer can be used. Preferably, the buffer is a tris buffer, phosphate buffer, carbonate buffer, sodium phosphate buffer, glycine sodium hydroxide buffer, sodium hydrogen phosphate buffer, sodium dihydrogen phosphate buffer, potassium phosphate buffer, potassium hydrogen phosphate buffer, potassium dihydrogen phosphate buffer, pyrophosphate buffer, sodium pyrophosphate/hydrochloric acid buffer, sodium carbonate buffer, potassium carbonate buffer, sodium hydrogen carbonate buffer, and/or potassium hydrogen carbonate buffer, more preferably wherein the buffer is a 10-100 mM tris buffer, even more preferably a 30-80 mM tris buffer, even more preferably a 40-60 mM tris buffer, and most preferably a 50 mM tris buffer.

Yet another embodiment pertains to the use of the second aspect of the present invention, wherein the extraction solution has an ionic strength corresponding to a sodium chloride concentration of at least 200 mM, preferably corresponding to a sodium chloride concentration of at least 300 mM, and most preferably corresponding to a sodium chloride concentration of around 400 mM.

The present invention pertains to the use of an ionic detergent having a steroid scaffold, for the extraction of any kind of allergen from a sample. In one embodiment, the allergen is a food allergen, preferably wherein the food allergen is a nut or legume allergen (e.g., from peanut, walnut, almond, pecan, cashew, hazelnut/filbert, pistachio, pine nut, or brazil nut), a dairy allergen (e.g., from egg, or milk), a seed allergen (e.g., from sesame, poppy, or mustard), a seafood allergen (e.g., from shrimp, fish, crab, lobster, clams, mussels, oysters, scallops, crayfish, or shellfish), soybean, wheat, or a fruit allergen (e.g., from strawberries, mango, banana, or apple).

According to the second aspect of present invention, an ionic detergent having a steroid scaffold can be used for the extraction of any kind of allergen from any kind of sample. In one embodiment, the sample is a biological allergen source material, such as a food sample, a sample derived from the environment, such as a sample from soil or from seawater, from at least one organism such as from at least one bacterium, fungus, protist, algae, plant, an animal, or a mixture thereof. Without being limited thereto, examples of food samples according to the present invention are nuts (such as peanuts, walnuts, almonds, pecans, cashews, hazelnuts, pistachios, pine nuts, or brazil nuts), eggs, milk, seeds (such as sesame, poppy, or mustard), seafood (such as shrimp, fish, crab, lobster, clams, mussels, oysters, scallops, crayfish, or shellfish), soybean, wheat, or fruits (such as strawberries, mango, banana, or apple). Importantly, the extraction solution of the present invention is able to extract allergens even from very fatty samples, such as peanut-containing food samples.

One embodiment relates to the above use of the second aspect of the present invention, wherein if the allergen is a nut or legume allergen or soy, the extraction solution preferably has an ionic strength corresponding to a sodium chloride concentration of at least 350 mM, even more preferably corresponding to a sodium chloride concentration of around 400 mM.

An ionic strength corresponding to a sodium chloride concentration of at least 350 mM is particularly preferred if the allergen is a nut or legume allergen (e.g. peanut), or soy, because globulins present in nuts, legumes, or soy are salt-soluble. However, many other allergens are also salt-soluble. Accordingly, it may be preferred that the extraction solution has an ionic strength corresponding to a sodium chloride concentration of at least 350 mM for any allergen to be extracted from a sample, to enable an efficient extraction from the sample. It is particularly preferred that the extraction solution has an ionic strength corresponding to a sodium chloride concentration of at least 350 mM for any allergen in globulin form to be extracted from a sample.

A further embodiment relates to the above use of the second aspect of this invention, wherein if the allergen comprises at least one disulfide bond, preferably more than one disulfide bonds, the extraction solution further comprises a reducing agent, such as Tris(2-carboxyethyl)phosphine (TCEP). Without being limited thereto, a further reducing agent that may be used is thioglycerol.

In one embodiment, the extraction solution does not comprise sodium dodecyl sulfate (SDS).

In a further embodiment, the extraction solution comprises 50 mM Tris pH 8.5, 400 mM sodium chloride, and 1% (w/v) sodium deoxycholate, or a derivate thereof.

A third aspect, which can be combined with any other aspect and specific embodiment of the present invention, pertains to a method for detecting an allergen in a sample, the method comprising the steps of:
a) Providing a sample, and an extraction solution comprising an ionic detergent having a steroid scaffold;
b) Contacting the sample with the extraction solution, and
c) Detecting the allergen in the sample.

According to the present invention, any kind of detection method can be used for detecting the allergen in the sample in step c) of the method. In one embodiment, the allergen is detected in step c) of the method using one or more antibodies specific for one or more allergens, such as by western blot, ELISA, or Proximity Extension Assay (PEA); and/or mass-spectrometrically, such as by liquid chromatography-multiple reaction monitoring/mass spectrometry (LC-MRM/MS).

Further detection methods that are preferred in the context of the herein described invention to detect the allergen are by means of a detection method such as mass spectrometry immunoassay (MSIA), antibody-based protein chips, 2-dimensional gel electrophoresis, stable isotope standard capture with anti-peptide antibodies (SISCAPA), high-performance liquid chromatography (HPLC), cytometry bead array (CBA), protein immuno-precipitation, radio immunoassay, and ligand binding assay. Suitable alternative detection methods for detection of an allergen of the invention are known to the skilled artisan.

In a preferred embodiment, the ionic detergent having a steroid scaffold is a bile acid, a bile acid derivative, a bile acid analog, or a salt thereof.

Further preferred is that the bile acid, the bile acid derivative, the bile acid analog, or the salt thereof is cholic acid, glycocholic acid, taurocholic acid, chenodeoxycholic acid, glycochenodeoxycholic acid, taurochenodeoxycholic acid, deoxycholate, sodium deoxycholate, deoxycholic acid, cholate, sodium cholate, deoxycholate, chenodeoxycholate, chenodeoxy cholic acid, ursodeoxycholic acid (UDCA), dehydrocholic acid, or sodium dehydrocholate, preferably wherein the ionic detergent is sodium deoxycholate, or a derivate thereof.

One embodiment relates to the method of the third aspect of the present invention, wherein the concentration of the ionic detergent having a steroid scaffold in the extraction solution is at least 0.01% (w/v) of the extraction solution, preferably at least 0.1% (w/v), and most preferably at least 1% (w/v) of the extraction solution.

In another preferred embodiment, the extraction solution further comprises a buffer, and wherein the pH of the extraction solution is between 7.0 - 10.0, preferably between 8.0 - 9.0, and most preferably around 8.5.

According to the present invention, any kind of buffer can be used. Preferably, the buffer is a tris buffer, phosphate buffer, carbonate buffer, sodium phosphate buffer, glycine sodium hydroxide buffer, sodium hydrogen phosphate buffer, sodium dihydrogen phosphate buffer, potassium phosphate buffer, potassium hydrogen phosphate buffer, potassium dihydrogen phosphate buffer, pyrophosphate buffer, sodium pyrophosphate/hydrochloric acid buffer, sodium carbonate buffer, potassium carbonate buffer, sodium hydrogen carbonate buffer, and/or potassium hydrogen carbonate buffer, more preferably wherein the buffer is a 10-100 mM tris buffer, even more preferably a 30-80 mM tris buffer, even more preferably a 40-60 mM tris buffer, and most preferably a 50 mM tris buffer.

Yet another embodiment pertains to the method of the third aspect of the present invention, wherein the extraction solution has an ionic strength corresponding to a sodium chloride concentration of at least 200 mM, preferably corresponding to a sodium chloride concentration of at least 300 mM, and most preferably corresponding to a sodium chloride concentration of around 400 mM.

In one embodiment, the contacting in step b) of the method is performed at a temperature of between 80°C and 120°C, preferably between 90°C and 110°C, more preferably between 95°C and 105°C, and most preferably at around 99°C. In a further preferred embodiment, the contacting in step b) of the method is performed for a duration of at least 10 min, preferably for at least 20 min, more preferably around 20 to 30 min.

The present invention enables extraction of any kind of allergen. In one embodiment, the allergen is a food allergen, preferably wherein the food allergen is a nut or legume allergen (e.g., from peanut, walnut, almond, pecan, cashew, hazelnut/filbert, pistachio, pine nut, or brazil nut), a dairy allergen (e.g., from egg, or milk), a seed allergen (e.g., from sesame, poppy, or mustard), a seafood allergen (e.g., from shrimp, fish, crab, lobster, clams, mussels, oysters, scallops, crayfish, or shellfish), soybean, wheat, or a fruit allergen (e.g., from strawberries, mango, banana, or apple).

According to the present invention, any kind of allergen can be extracted from any kind of sample, and subsequently be analyzed, i.e. detected. In one embodiment, the sample is a biological allergen source material, such as a food sample, a sample derived from the environment, such as a sample from soil or from seawater, from at least one organism such as from at least one bacterium, fungus, protist, algae, plant, an animal, or a mixture thereof. Examples of food samples according to the present invention are nuts (such as peanuts, walnuts, almonds, pecans, cashews, hazelnuts, pistachios, pine nuts, or brazil nuts), eggs, milk, seeds (such as sesame, poppy, or mustard), seafood (such as shrimp, fish, crab, lobster, clams, mussels, oysters, scallops, crayfish, or shellfish), soybean, wheat, or fruits (such as strawberries, mango, banana, or apple). Importantly, the extraction solution of the present invention is able to extract allergens even from very fatty samples, such as peanut-containing food samples.

One embodiment relates to the above method of the third aspect of this invention, wherein if the allergen is a nut or legume allergen or soy, the extraction solution has an ionic strength corresponding to a sodium chloride concentration of at least 350 mM, preferably corresponding to a sodium chloride concentration of around 400 mM.

An ionic strength corresponding to a sodium chloride concentration of at least 350 mM is particularly preferred if the allergen is a nut or legume allergen (e.g. peanut), or soy, because globulins present in nuts, legumes, or soy are salt-soluble. However, many other allergens are also salt-soluble. It may be preferred that the extraction solution has an ionic strength corresponding to a sodium chloride concentration of at least 350 mM for any allergen to be extracted from a sample, to enable an efficient extraction from the sample. It is particularly preferred that the extraction solution has an ionic strength corresponding to a sodium chloride concentration of at least 350 mM for any allergen in globulin form to be extracted from a sample.

A further embodiment relates to the above method of the third aspect of this invention, wherein if the allergen comprises at least one disulfide bond, preferably more than one disulfide bonds, the extraction solution further comprises a reducing agent, such as Tris(2-carboxyethyl)phosphine (TCEP). Without being limited thereto, a further reducing agent that maybe used is thioglycerol.

In one embodiment, the extraction solution does not comprise sodium dodecyl sulfate (SDS).

In a further embodiment, the extraction solution comprises 50 mM Tris pH 8.5, 400 mM sodium chloride, and 1% (w/v) sodium deoxycholate, or a derivate thereof.

The methods of the third aspect of this invention, are preferably *ex-vivo* or *in-vitro* methods.

A fourth aspect of the present invention, which can be combined with any other aspect and specific embodiment of the present invention, pertains to a kit for performing a method according to the first and/or third aspect of the present invention.

In one embodiment, the kit comprises:
a) an extraction solution comprising an ionic detergent having a steroid scaffold, and
b) one or more antibodies, or antigen binding fragments thereof, for the detection of at least one allergen.

The kit may further contain a container holding its ingredients, and/or instructions for use.

Yet another embodiment pertains to the kit, wherein the ionic detergent having a steroid scaffold is a bile acid, a bile acid derivative, a bile acid analog, or a salt thereof, preferably wherein the bile acid, the bile acid derivative, the bile acid analog, or the salt thereof is cholic acid, glycocholic acid, taurocholic acid, deoxycholic acid, chenodeoxycholic acid, glycochenodeoxycholic acid, taurochenodeoxycholic acid, deoxycholate, sodium deoxycholate, cholate, sodium cholate, deoxycholate, chenodeoxycholate, chenodeoxy cholic acid, ursodeoxycholic acid (UDCA), dehydrocholic acid, or sodium dehydrocholate, preferably wherein the ionic detergent is sodium deoxycholate, or a derivate thereof.

In one embodiment, the concentration of the ionic detergent having a steroid scaffold in the extraction solution is at least 0.01% (w/v) of the extraction solution, preferably at least 0.1% (w/v), and most preferably at least 1% (w/v) of the extraction solution.

Preferably, the extraction solution of the kit of the fourth aspect of this invention further comprises a buffer, wherein the buffer is a tris buffer, phosphate buffer, carbonate buffer, sodium phosphate buffer, glycine sodium hydroxide buffer, sodium hydrogen phosphate buffer, sodium dihydrogen phosphate buffer, potassium phosphate buffer, potassium hydrogen phosphate buffer, potassium dihydrogen phosphate buffer, pyrophosphate buffer, sodium pyrophosphate/hydrochloric acid buffer, sodium carbonate buffer, potassium carbonate buffer, sodium hydrogen carbonate buffer, and/or potassium hydrogen carbonate buffer, preferably wherein the buffer is a 10-100 mM tris buffer, more preferably a 30-80 mM tris buffer, even more preferably a 40-60 mM tris buffer, and most preferably a 50 mM tris buffer, and wherein the pH of the extraction solution is between 7.0 - 10.0, preferably between 8.0 - 9.0, and most preferably is around 8.5.

In one preferred embodiment pertaining to the kit according to the fourth aspect of this invention, the extraction solution has an ionic strength corresponding to a sodium chloride concentration of at least 200 mM, preferably corresponding to a sodium chloride concentration of at least 300 mM, and most preferably corresponding to a sodium chloride concentration of around 400 mM, and wherein if the allergen is a nut or legume allergen or soy, the extraction solution has an ionic strength corresponding to a sodium chloride concentration of at least 350 mM, preferably corresponding to a sodium chloride concentration of around 400 mM.

The kit according to the fourth aspect of this invention can be used to detect any kind of allergen. In one preferred embodiment, the allergen is a food allergen, preferably wherein the food allergen is a nut or legume allergen (e.g., from peanut, walnut, almond, pecan, cashew, hazelnut/filbert, pistachio, pine nut, or brazil nut), a dairy allergen (e.g., from egg, or milk), a seed allergen (e.g., from sesame, poppy, or mustard), a seafood allergen (e.g., from shrimp, fish, crab, lobster, clams, mussels, oysters, scallops, crayfish, or shellfish), soybean, wheat, or a fruit allergen (e.g., from strawberries, mango, banana, or apple).

According to the present invention, any kind of allergen can be extracted from any kind of sample using the kit according to the fourth aspect of this invention. In one embodiment, the sample is a biological allergen source material, such as a food sample, a sample derived from the environment, such as a sample from soil or from seawater, from at least one organism such as from at least one bacterium, fungus, protist, algae, plant, an animal, or a mixture thereof. Examples of food samples according to the present invention are nuts (such as peanuts, walnuts, almonds, pecans, cashews, hazelnuts, pistachios, pine nuts, or brazil nuts), eggs, milk, seeds (such as sesame, poppy, or mustard), seafood (such as shrimp, fish, crab, lobster, clams, mussels, oysters, scallops, crayfish, or shellfish), soybean, wheat, or fruits (such as strawberries, mango, banana, or apple). Importantly, the kit of the present invention is able to extract allergens even from very fatty samples, such as peanut-containing food samples.

Another preferred embodiment pertains to the kit according to the fourth aspect of this invention, wherein if the allergen comprises at least one disulfide bond, preferably more than one disulfide bond, the extraction solution further comprises a reducing agent, such as Tris(2-carboxyethyl)phosphine (TCEP). Without being limited thereto, a further reducing agent that maybe used is thioglycerol.

Another preferred embodiment pertains to the kit according to the fourth aspect of this invention, wherein the extraction solution of the kit does not comprise sodium dodecyl sulfate (SDS).

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES

The figures show:

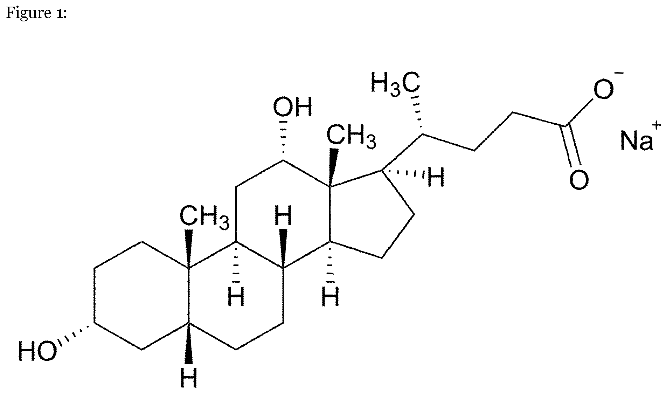

**Figure** 1 depicts the chemical structure of sodium deoxycholate.

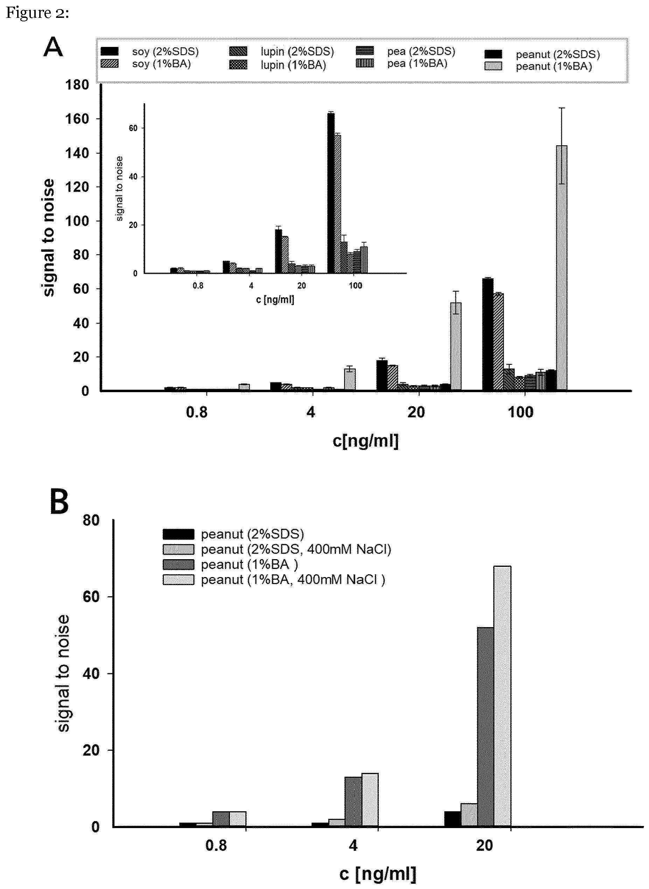

**Figure 2** shows the efficiency of the extraction of allergens from four different legume samples (soy, lupin, pea, and peanut). Allergens from samples were extracted using a method of the first aspect of this invention, i.e. using an extraction solution comprising an ionic detergent having a steroid scaffold, compared to state of the art methods using commonly used extraction solutions, and subsequently analyzed by multiplex analysis. Figure 2A shows the comparison of signal to noise ratios of the two different extraction methods (2% SDS compared to 1% ionic detergent having a steroid scaffold according to the present invention, e.g. a bile acid salt such as sodium deoxycholate (referred to as "BA"). No significant differences in extraction efficiencies are detectable in the case of soy, lupin and pea as shown in the enlarged insert of Figure 2A, showing that the extraction solution of this invention is as strong as the harshest denaturing solution SDS. Interestingly, peanut allergen solubilizes more than 10 times as efficient by the extraction solution of this invention. This extractability can be increased even further by addition of 400 mM NaCl as shown in Figure 2B, which depicts the comparison of 2% SDS + 400 mM NaCl to 1% ionic detergent having a steroid scaffold according to the present invention, e.g. a bile acid salt such as sodium deoxycholate (referred to as "BA") + 400 mM NaCl.

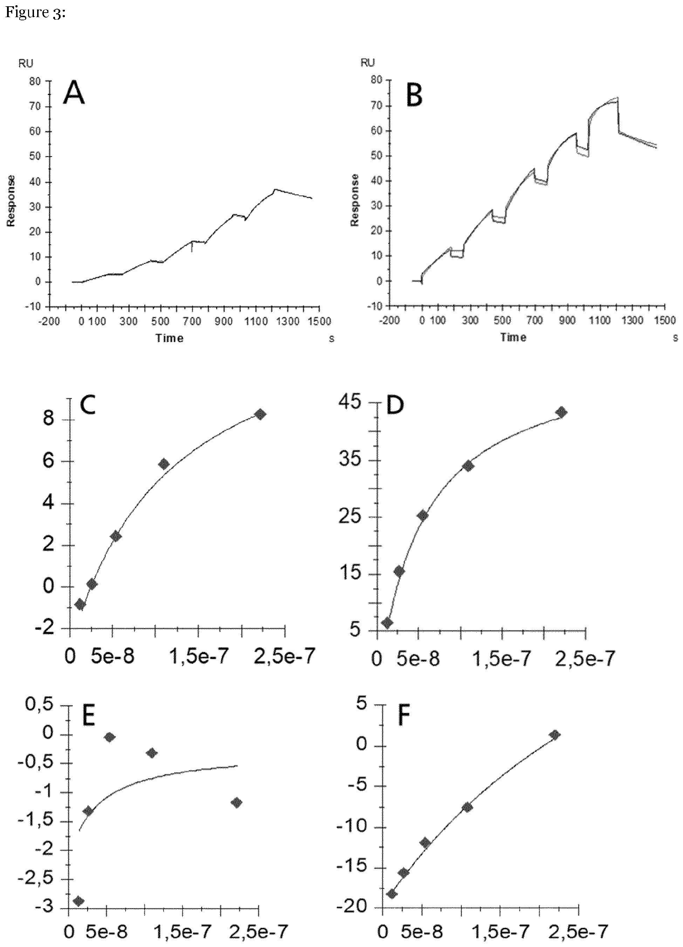

**Figure 3** depicts binding assays using Surface Plasmon Resonance spectroscopy (SPR) on a Biacore T200 instrument. The comparison between extraction solutions under denaturing conditions (A, C, E), compared to extraction of allergens using an extraction solution comprising an ionic detergent having a steroid scaffold according to the present invention, e.g. a bile acid salt such as sodium deoxycholate (B, D, F), is shown. Two different antibodies to be used as ligands which bind onto two different epitopes within the allergenic Gal d 2 protein are shown. Kinetic parameters of antibody-antigen binding were determined using the method single cycle kinetic (A, B). The specific Gal d 2-epitop denatured by the extraction solution (B) according to the present invention is significantly better accessible than the same epitope denatured by a classical denaturation solution (A) resulting in a significantly lower KD value of the analyte (Gal d 2). KD value in A is 1.42x10⁻⁸, KD value in B is 5.9x10⁻⁹. Affinity screens in C, D, E, and F illustrate the gain of accessibility of the two different epitopes of Gal d 2 too. The accessibility is achieved by denaturing of Gal d 2 with the extraction solution according to the present invention as shown in steady state affinities in D and F. In contrast, denaturation of Gal d 2 with a classical denaturation solution as shown in C and E lead to the low (C) or barely (E) accessibility of the epitope, as depicted by the corresponding affinity screens.

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Extraction of allergens using an extraction solution comprising an ionic detergent having a steroid scaffold

The inventors discovered that an extraction solution comprising an ionic detergent having a steroid scaffold, such as a bile acid, a bile acid derivative, a bile acid analog, or a salt thereof, is very effective in solubilizing and stabilizing an allergen during extraction. As such, the invention makes use of the emulsifying and solubilizing characteristics of bile acids and/or bile acid salts, which physiologically solubilize ingredients within the digestive tract.

The inventors further discovered that the presence of the ionic detergent having a steroid scaffold, such as a bile acid, a bile acid derivative, a bile acid analog, or a salt thereof, partly unfolds the allergen and makes it accessible for further detection reagents, such as antibodies used for detecting the allergen. In a partly unfolded state, an allergen is better accessible for the entry of antibodies, which allows a more efficient binding of antibodies to the allergen to be detected. Accordingly, the detection of the allergen is easier and more reliable when using a method of extracting an allergen according to the present invention.

It is also an advantage of the present invention that the extraction process is similar to what happens physiologically during the digestion of food. Accordingly, allergens extracted using the extraction method of the present invention have a physiological configuration, which is highly advantageous for further detection methods since allergens are in a state that would normally cause an allergic reaction within the consumer's body. Importantly, epitopes present within the allergen become accessible in presence of bile acid salts, such as sodium deoxycholate. This is true during physiological digestion, but also during in vitro extraction methods according to the present invention. Such epitopes are of great interest, since they can be the cause of an allergic reaction within the human body. As a comparison, other methods, such as those that rely on, e.g., addition of sodium dodecyl sulfate (SDS), extract allergens in a very artificial state that often largely differs from its physiological configuration.

The inventors discovered that any derivative of cholic acid in its ionic form and having ampiphatic character is particularly useful as ionic detergent in the sense of the present invention. Particularly preferred for extraction of an allergen is an ionic detergent having ampiphatic character, since the ampiphatic character enables a very efficient solubilization and stabilization of the allergen to be extracted from the sample.

Importantly, the inventors analysed that the novel extraction method according to the present invention extracts allergens more efficiently than a method using commonly used denaturating extraction solutions, such as those that are used for, e.g. 2D electrophoresis. One example of such denaturating extraction solutions are solutions that contain urea. Also, the inventors discovered that the addition of SDS to an extraction solution is not advantageous over an extraction solution comprising a bile acid, a bile acid derivative, a bile acid analog, or a salt thereof, such as cholic acid, glycocholic acid, taurocholic acid, chenodeoxycholic acid, glycochenodeoxycholic acid, taurochenodeoxycholic acid, deoxycholate, sodium deoxycholate, deoxycholic acid, cholate, sodium cholate, deoxycholate, chenodeoxycholate, chenodeoxy cholic acid, ursodeoxycholic acid (UDCA), dehydrocholic acid, or sodium dehydrocholate, preferably wherein the ionic detergent is sodium deoxycholate, or a derivate thereof.

Accordingly, the inventors' method allows manufacturers as well as certain agencies to analyze the contents of food in a reliable way, e.g. for the presence of allergens. The extraction methods of the present invention allow further analysis of allergens in a soluble state. Importantly, the soluble state of allergens is the requirement for reliably detecting the presence of allergens in a sample, e.g. by antibody-based methods and/or mass spectrometry.

### Example 2: Comparison of the efficiency of extraction of allergens using a denaturing solution comprising SDS, compared to the extraction solution according to the present invention

The inventors compared the efficiency of the extraction of allergens from four different legume samples (soy, lupin, pea, and peanut) of a commonly used denaturing solution comprising SDS, compared to the extraction solution comprising an ionic detergent having a steroid scaffold according to the present invention, e.g. a bile acid salt such as sodium deoxycholate. No significant differences in extraction efficiencies were detectable in the case of soy, lupin and pea as shown in the enlarged insert of Figure 2A, demonstrating that the extraction solution of this invention is as strong as the harshest denaturing solution SDS. Interestingly, the inventors observed that peanut allergen solubilizes more than 10 times as efficient when using the extraction solution according to this invention compared to a solution comprising SDS. The inventors further observed that this extractability can be increased even further by addition of 400 mM NaCl, as shown in Figure 2B.

Next, the inventors performed binding assays using Surface Plasmon Resonance spectroscopy (SPR) on a Biacore T200 instrument to determine kinetic parameters of antibody-antigen binding. The inventors compared extraction of allergens by an extraction solution under denaturing conditions (Fig. 3 A, C, E), to an extraction solution comprising an ionic detergent having a steroid scaffold according to the present invention, e.g. a bile acid salt such as sodium deoxycholate (Fig. 3 B, D, F). The inventors observed that when using the extraction solution according to the present invention (Fig. 3 B), the specific Gal d 2-epitop was significantly better accessible than the same epitope denatured by a classical denaturation solution (Fig. 3 A), resulting in a significantly lower KD value of the analyte (Gal d 2). The inventors determined that the KD value in Fig. 3 A is 1.42x10⁻⁸ (achieved by an extraction solution comprising SDS) and the KD value in Fig. 3 B is 5.9x10⁻⁹ (achieved by an extraction solution according to the present invention.) Affinity screens in Fig. 3 C, D, E, and F illustrate the gain of accessibility of the two different epitopes of Gal d 2. The accessibility is achieved by denaturing of Gal d 2 with the extraction solution according to the present invention as shown in steady state affinities in Fig. 3 D and Fig. 3 F. In contrast, denaturation of Gal d 2 with a classical denaturation solution as shown in Fig. 3 C and Fig. 3 E lead to the low (Fig. 3 C) or barely (Fig. 3 E) accessibility of the epitope, as depicted by the corresponding affinity screens.

## Claims

1. **A method** for extracting an allergen from a sample, the method comprising the steps of:
a) Providing a sample, and an extraction solution comprising an ionic detergent having a steroid scaffold, and
b) Contacting the sample with the extraction solution,
thereby extracting the allergen from the sample.

2. The method according to claim 1, wherein the ionic detergent having a steroid scaffold is a bile acid, a bile acid derivative, a bile acid analog, or a salt thereof, preferably wherein the bile acid, the bile acid derivative, the bile acid analog, or the salt thereof is cholic acid, glycocholic acid, taurocholic acid, chenodeoxycholic acid, glycochenodeoxycholic acid, taurochenodeoxycholic acid, deoxycholate, sodium deoxycholate, deoxycholic acid, cholate, sodium cholate, deoxycholate, chenodeoxycholate, chenodeoxy cholic acid, ursodeoxycholic acid (UDCA), dehydrocholic acid, or sodium dehydrocholate, more preferably wherein the ionic detergent is sodium deoxycholate, or a derivate thereof.

3. The method according to claim 1 or 2, wherein the concentration of the ionic detergent having a steroid scaffold in the extraction solution is at least 0.01% (w/v) of the extraction solution, preferably at least 0.1% (w/v), and most preferably at least 1% (w/v) of the extraction solution.

4. The method according to any one of the preceding claims, wherein the extraction solution further comprises a buffer selected from a tris buffer, phosphate buffer, carbonate buffer, sodium phosphate buffer, glycine sodium hydroxide buffer, sodium hydrogen phosphate buffer, sodium dihydrogen phosphate buffer, potassium phosphate buffer, potassium hydrogen phosphate buffer, potassium dihydrogen phosphate buffer, pyrophosphate buffer, sodium pyrophosphate/hydrochloric acid buffer, sodium carbonate buffer, potassium carbonate buffer, sodium hydrogen carbonate buffer, and potassium hydrogen carbonate buffer, preferably wherein the buffer is a 10-100 mM tris buffer, more preferably a 30-80 mM tris buffer, even more preferably a 40-60 mM tris buffer, and most preferably a 50 mM tris buffer, and wherein the pH of the extraction solution is between 7.0 - 10.0, preferably between 8.0 - 9.0, and most preferably around 8.5.

5. The method according to any one of the preceding claims, wherein the extraction solution has an ionic strength corresponding to a sodium chloride concentration of at least 200 mM, preferably corresponding to a sodium chloride concentration of at least 300 mM, and most preferably corresponding to a sodium chloride concentration of around 400 mM, and wherein if the allergen is a nut allergen, legume allergen or soy, the extraction solution preferably has an ionic strength corresponding to a sodium chloride concentration of at least 350 mM, more preferably corresponding to a sodium chloride concentration of around 400 mM.

6. The method according to any one of the preceding claims, wherein the contacting in step b) is performed at a temperature of between 80°C and 120°C, preferably between 90°C and 110°C, more preferably between 95°C and 105°C, and most preferably at around 99°C.

7. The method according to any one of the preceding claims, wherein the allergen is a food allergen, preferably wherein the food allergen is a nut or legume allergen (e.g., from peanut, walnut, almond, pecan, cashew, hazelnut/filbert, pistachio, pine nut, or brazil nut), a dairy allergen (e.g., from egg, or milk), a seed allergen (e.g., from sesame, poppy, or mustard), a seafood allergen (e.g., from shrimp, fish, crab, lobster, clams, mussels, oysters, scallops, crayfish, or shellfish), soybean, wheat, or a fruit allergen (e.g., from strawberries, mango, banana, or apple).

8. The method according to any one of the preceding claims, wherein the sample is a biological allergen source material, such as a food sample, a sample derived from the environment, such as a sample from soil or from seawater, from at least one organism such as from at least one bacterium, fungus, protist, algae, plant, an animal, or a mixture thereof, preferably wherein the food sample is a sample from nuts (such as peanuts, walnuts, almonds, pecans, cashews, hazelnuts, pistachios, pine nuts, or brazil nut), eggs, milk, seeds (such as sesame, poppy, or mustard), seafood (such as shrimp, fish, crab, lobster, clams, mussels, oysters, scallops, crayfish, or shellfish), soybean, wheat, or fruits (such as strawberries, mango, banana, or apple).

9. The method according to any one of the preceding claims, wherein if the allergen comprises at least one disulfide bond, or more than one disulfide bonds, the extraction solution further comprises a reducing agent, such as Tris(2-carboxyethyl)phosphine (TCEP).

10. The method according to any one of the preceding claims, wherein the extraction solution does not comprise sodium dodecyl sulfate (SDS), optionally wherein the extraction solution comprises 50 mM Tris pH 8.5, 400 mM sodium chloride, and 1% (w/v) sodium deoxycholate, or a derivate thereof.

11. **Use** of an ionic detergent having a steroid scaffold, wherein the ionic detergent having a steroid scaffold is a bile acid, a bile acid derivative, a bile acid analog, or a salt thereof, for the extraction of an allergen from a sample.

12. **A method** for detecting an allergen in a sample, the method comprising the steps of:
a) Providing a sample, and an extraction solution comprising an ionic detergent having a steroid scaffold;
b) Contacting the sample with the extraction solution, and
c) Detecting the allergen in the sample.

13. The method according to claim 12, wherein the allergen is detected in step c) of the method using one or more antibodies specific for one or more allergens, such as by western blot, ELISA, or Proximity Extension Assay (PEA); and/or mass-spectrometrically, such as by liquid chromatography-multiple reaction monitoring/mass spectrometry (LC-MRM/MS).

14. **A kit** for performing a method according to any one of the preceding claims.

15. The kit according to claim 14, comprising
(i) an extraction solution comprising an ionic detergent having a steroid scaffold, preferably wherein the ionic detergent having a steroid scaffold is a bile acid, a bile acid derivative, a bile acid analog, or a salt thereof, and
(ii) one or more antibodies, or antigen binding fragments thereof, for the detection of at least one allergen.
